(19) Europäisches Patentamt
European Patent Office
Office européen des brevets

(11) EP 1 454 899 A1

(12) **EUROPEAN PATENT APPLICATION**
published in accordance with Art. 158(3) EPC

(43) Date of publication:
**08.09.2004 Bulletin 2004/37**

(21) Application number: **02777853.9**

(22) Date of filing: **16.10.2002**

(51) Int Cl.[7]: **C07D 213/30**

(86) International application number:
**PCT/JP2002/010730**

(87) International publication number:
**WO 2003/033468 (24.04.2003 Gazette 2003/17)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **17.10.2001 JP 2001319643**

(71) Applicant: **KANEKA CORPORATION**
**Osaka-shi, Osaka 530-8288 (JP)**

(72) Inventors:
- **TAOKA, Naoaki, c/o KANEKA CORPORATION**
  **Takasago-shi, Hyogo 676-8688 (JP)**
- **KIZAKI, Noriyuki, c/o KANEKA CORPORATION**
  **Takasago-shi, Hyogo 676-8688 (JP)**
- **KAWANO, Shigeru,**
  **c/o KANEKA CORPORATION**
  **Takasago-shi, Hyogo 676-8688 (JP)**
- **HORIKAWA, Miho, c/o KANEKA CORPORATION**
  **Takasago-shi, HYogo 676-8688 (JP)**
- **YASOHARA, Yoshihiko,**
  **c/o KANEKA CORPORATION**
  **Takasago-sHi, Hyogo 676-8688 (JP)**

(74) Representative: **VOSSIUS & PARTNER**
**Siebertstrasse 4**
**81675 München (DE)**

(54) **PROCESS FOR PREPARATION OF (S)-ALPHA-HALOME THYLPYRIDINE METHANOL DERIVATIVES**

(57) The present invention provides a process for easily preparing a (S)-α-halomethylpyridine-methanol derivative, which is useful as an intermediate of pharmaceutical products, from inexpensive raw materials. The (S)-α-halomethylpyridine-methanol derivative is prepared by (S)-selectively reducing a 2-haloacetylpyridine derivative using an enzyme source having ability to (S)-selectively reduce a carbonyl group of the 2-haloacetylpyridine derivative, which can be obtained inexpensively. Also, a hydrohalic acid salt of (S)-α-halomethyl-3-pyridine-methanol derivative is isolated and purified as crystal from a (S)-α-halomethyl-3-pyridine-methanol derivative containing impurities using hydrohalic acid and an organic solvent.

EP 1 454 899 A1

## Description

TECHNICAL FIELD

The present invention relates to a process for preparing a (S)-α-halomethylpyridine-methanol derivative, particularly a (S)-α-chloromethyl-3-pyridine-methanol derivative, which is useful as an intermediate of pharmaceutical products. Specifically, the present invention relates to a process for preparing a (S)-α-halomethylpyridine-methanol derivative, particularly a (S)-α-chloromethyl-3-pyridine-methanol derivative, which comprises (S)-selectively reducing a 2-haloacetylpyridine derivative using an enzyme having ability to (S)-selectively reduce a carbonyl group of the 2-haloacetylpyridine derivative.

BACKGROUND ART

The (S)-α-halomethyl-3-pyridine-methanol derivative and the hydrohalic acid salt thereof of the present invention are new compounds, which are not yet disclosed.
The following processes are conventionally known as processes for preparing substances similar to the compound of the present invention.

1. The process of preparing methyl (S)-6-(2-bromo-1-hydroxyethyl)pyridine carboxylate by reducing methyl 6-bromoacetylpyridine-2-carboxylate using chiral oxazoborolidine (Tetrahedron, 53 (37), 12405-12414, (1997))
2. The process of preparing methyl (S)-6-(2-chloro-1-hydroxyethyl)pyridine carboxylate by reducing methyl 6-chloroacetylpyridine-2-carboxylate using a microorganism belonging to Geotrichum genus (Tetrahedron, 54 (43), 13059-13072, (1998)).

However, in process 1, an expensive reducing agent is necessary and the optical purity of the product is not high. In process 2, productivity is low, as the substrate concentration is low, and the optical purity of the product is not high. In this way, both of the conventional methods are extremely problematic for industrial use and for application to the compound of the present invention, which is a new compound.
In view of the above, the object of the present invention is to provide a process for easily preparing a (S)-α-halomethylpyridine-methanol derivative, particularly a (S)-α-halomethyl-3-pyridine-methanol derivative, which is useful as an intermediate of pharmaceutical products, using inexpensive and easily-obtainable raw materials.

DISCLOSURE OF INVENTION

As a result of intensive studies in light of the above, a process was found for efficiently preparing a (S)-α-halomethylpyridine-methanol derivative by (S)-selectively reducing a 2-haloacetylpyridine derivative using an enzyme having ability to (S)-selectively reduce a carbonyl group of the 2-haloacetylpyridine derivative, which can be obtained inexpensively. Thus, the present invention was achieved.
That is, the present invention relates to a (S)-α-halomethyl-3-pyridine-methanol derivative represented by formula (1):

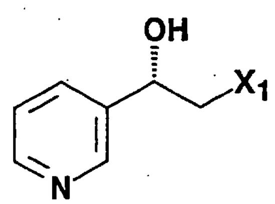

(1)

(wherein $X_1$ represents a halogen atom).
$X_1$ is preferably a chlorine atom.
Also, the present invention relates to a hydrohalic acid salt of a (S)-α-halomethyl-3-pyridine-methanol derivative represented by formula (2):

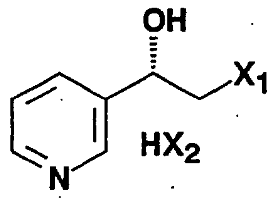

(2)

(wherein $X_1$ and $X_2$ represent a halogen atom).

$X_1$ and $X_2$ are preferably a chlorine atom.

The present invention also relates to a process for preparing a (S)-α-halomethylpyridine-methanol derivative represented by formula (4):

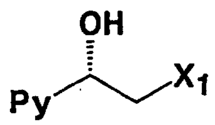

(4)

(wherein $X_1$ represents a halogen atom), which comprises (S)-selectively reducing a 2-haloacetylpyridine derivative represented by formula (3):

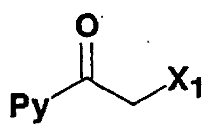

(3)

(wherein $X_1$ represents a halogen atom and Py represents a pyridyl group which can have a substituent) using an enzyme having ability to (S)-selectively reduce a carbonyl group of the 2-haloacetylpyridine derivative.

$X_1$ is preferably a chlorine atom.

Py is preferably a 3-pyridyl group, which can have a substituent.

The substituent of the pyridyl group is preferably at least one member selected from the group consisting of a nitro group, an N-protected amino group, a halogen atom and an alkoxy group having 1 to 10 carbon atoms.

The reducing enzyme having ability to (S)-selectively reduce a carbonyl group of the 2-haloacetylpyridine derivative is preferably an enzyme present in a culture or a treated substance of a microorganism selected from the group consisting of Candida genus, Clavispora genus, Cryptococcus genus, Debaryomyces genus, Geotrichum genus, Hyphopichia genus, Metschnikowia genus, Ogataea genus, Pachysolen genus, Pichia genus, Rhodotorula genus, Saccharomyces genus, Trichosporon genus, Trigonopsis genus, Waltomyces genus, Yamadazyma genus and Yarrowia genus; and/or a purified reducing enzyme derived from the microorganism.

The enzyme having ability to (S)-selectively reduce a carbonyl group of the 2-haloacetylpyridine derivative is preferably an enzyme present in a culture or a treated substance of a microorganism selected from the group consisting of Candida etchellsii IFO 1229, Candida etchellsii IFO 1942, Candida galacta IFO 10031, Candida lactis-condensi IFO 1286, Candida versatilis IFO 1941, Candida fennica CBS 6028, Candida magnoliae IFO 0705, Candida maris IFO 10003, Clavispora lusitaniae IFO 1019, Cryptococcus humicola CBS 5958, Debaryomyces carsonii IFO 0795, Debaryomyces hansenii var. hansenii IFO 0795, Geotrichum candidum CBS 187.67, Hyphopichia burtonii IFO 0844, Metschnikowia pulcherrima IFO 0561, Ogataea polymorpha IFO 1475, Pachysolen tannophilus IFO 1007, Pichia anomala IFO 0707, Rhodotorula araucariae IFO 10053, Rhodotorula minuta IFO 0928, Saccharomyces bayanus IFO 0676, Trichosporon aquatile ATCC 22310, Trigonopsis variabilis IFO 0671, Waltomyces lipofer IFO 0673, Yamadazyma farinosa IFO 0459 and Yarrowia lipolytica IFO 1542; and/or a purified reducing enzyme derived from the microorganism.

The enzyme having ability to (S)-selectively reduce a carbonyl group of the 2-haloacetylpyridine derivative is preferably an enzyme present in a culture or a treated substance of a transformant of a microorganism selected from the group consisting of Candida genus, Clavispora genus, Cryptococcus genus, Debaryomyces genus, Geotrichum

genus, Hyphopichia genus, Metschnikowia genus, Ogataea genus, Pachysolen genus, Pichia genus, Rhodotorula genus, Saccharomyces genus, Trichosporon genus, Trigonopsis genus, Waltomyces genus, Yamadazyma genus and Yarrowia genus, which is transformed by a gene of a reducing enzyme.

The transformant is preferably Escherichia coli HB101 (pNTS1G) accession number PERM BP-5835 (February 24, 1997, International Patent Organism Depositary, National Institute of Advanced Industrial Science and Technology, address: AIST Tsukuba Central 6, 1-1, Higashi 1-Chome, Tsukuba-shi, Ibaraki-ken 305-8566. Japan) or Escherichia coli HB101 (pNTFPG) accession number FERM BP-7117 (April 11, 2000, International Patent Organism Depositary, National Institute of Advanced Industrial Science and Technology, address: AIST Tsukuba Central 6, 1-1, Higashi 1-Chome, Tsukuba-shi, Ibaraki-ken 305-8566 Japan).

The present invention also relates to a process for isolating and purifying, which comprises obtaining the compound represented by formula (1):

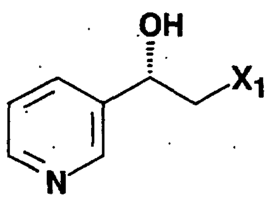

(1)

(wherein $X_1$ represents a halogen atom) as crystal of a hydrohalic acid salt of a (S)-α-halomethyl-3-pyridine-methanol derivative represented by formula (2):

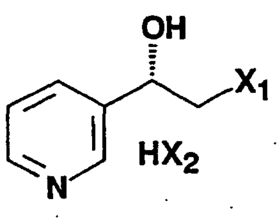

(2)

(wherein $X_1$ and $X_2$ represent a halogen atom) from a solution containing the (S)-α-halomethyl-3-pyridine-methanol derivative represented by formula (1) and impurities using a hydrohalic acid salt represented by formula (5):

$$X_2H \quad (5)$$

(wherein $X_2$ represents a halogen atom) and an organic solvent.

The (S)-α-halomethyl-3-pyridine-methanol derivative represented by formula (1) is preferably prepared by the above process.

$X_1$ is preferably a chlorine atom.

$X_2$ is preferably a chlorine atom.

The impurities are preferably enantiomers of the (S)-α-halomethyl-3-pyridine-methanol derivative represented by formula (1).

The organic solvent is preferably at least one member selected from the group consisting of a hydrocarbon solvent, an ester solvent, an ether solvent, a ketone solvent, a nitrile solvent, a halogen solvent and an alcohol.

The organic solvent is at least one member selected from the group consisting of pentane, hexane, cyclohexane, methylcyclohexane, heptane, octane, isooctane, benzene, toluene, ethylbenzene, propylbenzene, o-xylene, m-xylene, p-xylene, methyl acetate, ethyl acetate, n-propyl acetate, n-butyl acetate, tert-butyl acetate, methyl propionate, tert-butyl methyl ether, diethyl ether, diisopropyl ether, di-n-butyl ether, tetrahydrofurane, 1,4-dioxane, anisole, acetone, methyl ethyl ketone, diethyl ketone, acetonitrile, propionitrile, methylene chloride, chloroform, carbon tetrachloride, 1,2-dichloroethane, chlorobenzene, methanol, ethanol, n-propanol, isopropanol and n-butanol.

BEST MODE FOR CARRYING OUT THE INVENTION

The present invention is described in detail below.

In the above formulas (1), (2), (3) and (4), $X_1$ represents a halogen atom such as a fluorine atom, a chlorine atom, a bromine atom and an iodine atom. From the viewpoints of economical efficiency and ease in handling, $X_1$ preferably represents a chlorine atom.

In the above formulas (2) and (5), $X_2$ represents a halogen atom such as a fluorine atom, a chlorine atom, a bromine atom and an iodine atom. From the viewpoints of economical efficiency and ease in handling, $X_2$ preferably represents a chlorine atom.

In the 2-haloacetylpyridine derivative and the (S)-$\alpha$-halomethylpyridine-methanol derivative represented by formulas (3) and (4), Py represents a pyridyl group, which can have a substituent. Examples of the pyridyl group are a 2-pyridyl group, a 3-pyridyl group and a 4-pyridyl group and of these, a 3-pyridyl group is preferable. Examples of the substituent of the pyridyl group are halogen atoms such as a fluorine atom, a chlorine atom, a bromine atom and an iodine atom, nitro groups, nitroso groups, cyano groups, amino groups, hydroxyamino groups, alkylamino groups having 1 to 10 carbon atoms, dialkylamino groups having 1 to 10 carbon atoms, N-protected amino groups, azido groups, trifluoromethyl groups, carboxyl groups, formyl groups, acetyl groups, benzoyl groups, hydroxyl groups, alkoxy groups having 1 to 10 carbon atoms, acyloxy groups having 1 to 10 carbon atoms and alkyl thio groups having 1 to 10 carbon atoms. In view of practicability, nitro groups, amino groups, N-protected amino groups, halogen atoms and alkoxy groups having 1 to 10 carbon atoms are preferable. The number of substituents is preferably 0 to 3.

Herein, an example of a protective group of an N-protected amino group is the protective group described in pages 309 to 384 of "Protective Groups in Organic Synthesis, 2nd. Ed.", Theodora W. Green, published by John Wiley & Sons, 1990. Specific examples are aralkyl protective groups such as a benzyl group, a phenethyl group and a triphenylmethyl group; sulfonyl protective groups such as a methanesulfonyl group, a benzenesulfonyl group, a p-toluenesulfonyl group, an o-nitrobenzenesulfonyl group, a m-nitrobenzenesulfonyl group, a p-nitrobenzenesulfonyl group and a trifluoromethanesulfonyl group; carbamate protective groups such as a methoxycarbonyl group, an ethoxycarbonyl group, a benzyloxycarbonyl group and a tert-butoxycarbonyl group; and acetyl protective groups such as a phthaloyl group, an acetyl group, a chloroarotyl group, a trifluoroacetyl group, a pivaloyl group and a benzoyl group. Of these, an acetyl group is preferable.

The (S)-$\alpha$-halomethyl-3-pyridine-methanol derivative represented by formula (1) and the hydrohalic acid salt of the (S)-$\alpha$-halomethyl-3-pyridine-methanol derivative represented by formula (2) are new compounds, which are not yet disclosed.

The 2-haloacetylpyridine derivative represented by formula (3) can easily be synthesized by halogenating an acetylpyridine derivative using a halogenating agent. For example, 3-(2-chloroacetyl)-pyridine can easily be synthesized by chlorinating 3-acetylpyridine, which can be obtained inexpensively, using N-chlorosuccinimide (NCS) (see JP-A-9-512275).

The process for preparing the (S)-$\alpha$-halomethylpyridine-methanol derivative of the present invention is described below.

In the present invention, the (S)-$\alpha$-halomethylpyridine-methanol derivative represented by formula (4) is prepared by (S)-selectively reducing the carbonyl group of the 2-haloacetylpyridine derivative represented by formula (3), in the presence of an enzyme having ability to (S)-selectively reduce a carbonyl group of the 2-haloacetylpyridine derivative represented by formula (3).

Examples of the enzyme having ability to (S)-selectively reduce a carbonyl group of the 2-haloacetylpyridine derivative that is used in the reducing step are reductase and dehydrogenase. Specific examples of the enzyme are enzymes derived from a microorganism selected from the group consisting of Candida genus, Clavispora genus, Cryptococcus genus, Debaryomyces genus, Geotrichum genus, Hyphopichia genus, Metschnikowia genus, Ogataea genus, Pachysolen genus, Pichia genus, Rhodotorula genus, Saccharomyces genus, Trichosporon genus, Trigonopsis genus, Waltomyces genus, Yamadazyma genus and Yarrowia genus.

Preferable enzymes are enzymes derived from Candida etchellsii IFO 1229, Candida etchellsii IFO 1942, Candida galacta IFO 10031, Candida lactis-condensi IFO 1286, Candida versatilis IFO 1941, Candida fennica CBS 6028, Candida magnoliae IFO 0705, Candida maris IFO 10003, Clavispora lusitaniae IFO 1019, Cryptococcus humicola CBS 5958, Debaryomyces carsonii IFO 0795, Debaryomyces hansenii var. hansenii IFO 0795, Geotrichum candidum CBS 187.67, Hyphopichia burtonii IFO 0844, Metschnikowia pulcherrima IFO 0561, Ogataea polymorpha IFO 1475, Pachysolen tannophilus IFO 1007, Pichia anomala IFO 0707, Rhodotorula araucariae IFO 10053, Rhodotorula minuta IFO 0928, Saccharomyces bayanus IFO 0676, Trichosporon aquatile ATCC 22310, Trigonopsis variabilis IFO 0671, Waltomyces lipofer IFO 0673, Yamadazyma farinosa IFO 0459 and Yarrowia lipolytica IFO 1542.

As the enzyme, a purified reducing enzyme derived from the above microorganism, as well as an enzyme present in a culture or a treated substance of the above microorganism, can be used. In the case of the latter, the enzyme present in the culture or the treated substance can be used without purifying the culture or the treated substance. Herein, "a culture of a microorganism" refers to a culture solution containing a microorganism or a cultured microorganism and "a treated substance of a microorganism" refers to, for example, a crude extract, a freeze-dried microorganism, an acetone-dried microorganism or a fracture of the microorganism. Furthermore, these can be used by immobilizing the enzyme itself by a known method or by immobilizing as a microorganism by a known method. Immobilization can be conducted by a method known to one skilled in the art (such as the crosslinking method, the physical absorption method and the inclusion method).

As the above microorganism, both a wild strain and a mutant strain can be used and a microorganism derived from a genetic method, such as cell fusion or genetic engineering, can be used. The genetically engineered microorganism, which produces the enzyme used in the present invention, can be obtained by a known method. An example of the method that can be used is the method comprising a step of isolating and/or purifying the enzyme used in the present invention to determine part or all of the amino acid sequence of the enzyme, a step of obtaining the DNA sequence that encodes the enzyme based on the amino acid sequence, a step of obtaining a recombinant microorganism by introducing the DNA into another microorganism and a step of culturing the recombinant microorganism to obtain the enzyme used in the present invention (see WO98/35025).

The medium for the microorganism used as the enzyme source is not particularly limited, as long as the microorganism can proliferate. For example, the medium that can be used is a common liquid medium containing a carbon source such as saccharides including glucose and sucrose, alcohols including ethanol and glycerol, aliphatic acids including oleic acid and stearic acid and esters thereof and oils including rapeseed oil and soya bean oil; a nitrogen source such as ammonium sulfate, sodium nitrate, peptone, casamino acid, corn-steep liquor, bran and yeast extract; an inorganic salt such as magnesium sulfate, sodium chloride, calcium carbonate, potassium monohydrogenphosphate and potassium dihydrogenphosphate; or another nutrition source such as malt extract and meat extract. Culturing is conducted aerobically and usually the culture time is approximately 1 to 5 days, the pH of the medium is 3 to 9 and the culture temperature is 10 to 50°C. When the pH is less than 3 or more than 9 or the temperature is lower than 10°C or higher than 50°C, depending on the microorganism to be cultured, the microorganism may not proliferate or the proliferation rate may be extremely slow.

In the reduction process of the present invention, the reaction is conducted by adding the 2-haloacetylpyridine derivative, which is the substrate, a coenzyme NAD(P)H and the above enzyme source to a suitable solvent and then stirring while adjusting the pH. The reaction conditions depend on the enzyme, the microorganism or treated substance thereof that are used and the substrate concentration. Usually, the substrate concentration is approximately 0.1 to 100 % by weight, preferably 1 to 60 % by weight and the amount of the coenzyme NAD(P)H is 0.0001 to 100 % by mol, preferably 0.0001 to 0.1 % by mol, based on the substrate. The reaction temperature is 10 to 60°C, preferably 20 to 50°C, the pH of the reaction is 4 to 9, preferably 5 to 8, and the reaction time is 1 to 120 hours, preferably 1 to 72 hours. The substrate can be added all at once or continuously. The reaction can be conducted by a batch method or by a continuous method.

In the reduction process of the present invention, by using in combination with a commonly used coenzyme NAD(P)H regeneration system, the amount of the expensive coenzyme that is used can be reduced significantly. A typical example of the NAD(P)H regeneration system is the method of using glucose dehydrogenase and glucose.

In the case that the reaction is conducted in the same manner as above using a culture or a treated substance of a transformed microorganism, wherein both a gene of a reducing enzyme and a gene of an enzyme having ability to regenerate the coenzyme on which this enzyme depends (for example glucose dehydrogenase) are introduced into the host microorganism, a (S)-2-halomethylpyridine-methanol derivative can be prepared at lower cost, as the enzyme necessary for regeneration of the coenzyme does not need to be prepared separately.

An example of the transformed microorganism is a transformed cell, which is transformed by a plasmid comprising DNA that encodes the reducing enzyme used in the present invention and DNA that encodes the enzyme having ability to regenerate the coenzyme on which this enzyme depends.

An example of DNA that encodes the reducing enzyme used in the present invention is a gene of a reducing enzyme derived from a microorganism described above. Genes of a reducing enzyme derived from Candida magnoliae IFO 0705 and Candida maris IFO 10003 are preferable. The genes of these enzymes are suitable for use in the present invention, from the viewpoint that activity, selectivity and stability of the enzyme that the gene encodes are high. An example of DNA that encodes the enzyme having ability to regenerate the coenzyme is preferably a gene of glucose dehydrogenase and a gene of glucose dehydrogenase derived from Bacillus megaterium. This gene is suitable for use in the present invention, from the viewpoint that activity and stability of the enzyme that the gene encodes are high. Examples of the plasmid used for transformation are pNTS1G and pNTFPG. These plasmids are suitable for use in the present invention, from the viewpoint that the gene by which these plasmids are comprised can be overexpressed. The host cell is not particularly limited, as long as a microorganism that expresses the reducing enzyme and the enzyme having ability to regenerate the coenzyme is used, and Escherichia coli is preferable. Escherichia coli is suitable for use in the present invention, from the viewpoints that Escherichia coli can be cultured easily and be safely used industrially.

Examples of the transformed cell are Escherichia coli HB101 (pNTS1G) and Escherichia coli HB101 (pNTFPG). Escherichia coli HB101 (pNTS1G) was deposited as accession number FERM BP-5835 on February 24, 1997 and Escherichia coli HB101 (pNTFPG) was deposited as accession number FERM BP-7117 on April 11, 2000 to International Patent Organism Depositary, National Institute of Advanced Industrial Science and Technology (address: AIST Tsukuba Central 6, 1-1, Higashi 1-Chome, Tsukuba-shi, Ibaraki-ken 305-8566 Japan).

The reduction process of the present invention can be conducted in combination with a coenzyme regeneration system. In the case that an enzyme present in a culture or a treated substance of the above transformed microorganism is used, the reaction can be conducted using oxidized $NAD(P)^+$, which is less expensive, as the coenzyme.

The (S)-α-halomethylpyridine-methanol derivative produced in the reduction reaction can be purified by the usual method. For example, (S)-α-chloromethyl-3-pyridine-methanol can be purified by removing suspended substances such as cells by subjecting to treatment such as centrifugation and filtration according to need when a microorganism is used, then extracting by an organic solvent such as ethyl acetate and toluene, removing the organic solvent under reduced pressure and conducting treatment such as chromatography.

The process for isolating and purifying the (S)-α-halomethyl-3-pyridine-methanol derivative represented by formula (1) in the present invention is described below.

The (S)-α-halomethyl-3-pyridine-methanol derivative represented by formula (1), which is prepared by (S)-selectively reducing a carbonyl group of a 3-(2-haloacetyl)-pyridine derivative in the presence of an enzyme derived from a microorganism, tends to contain various impurities, as a result of various decompositions and side reactions in the preparation process. Particularly, in the reduction reaction by an enzyme having low stereo-recognizing ability of a carbonyl group of a 3-(2-haloacetyl)-pyridine derivative, a large amount of enantiomers of compound (1) tend to be by-produced and in order to obtain a high quality object, such impurities must be removed. Usually, removing impurities having similar structure (related substances) is difficult and in order to remove these impurities and obtain a high quality object, an excellent process for purifying and isolating is necessary. As a result of intensive studies, the present inventors have developed a process for efficiently obtaining an object of high purity, wherein impurities are removed after compound (1) ((S)-α-halomethyl-3-pyridine-methanol derivative), which contains impurities, is converted to hydrohalic acid salt of (S)-α-halomethyl-3-pyridine-methanol derivative represented by formula (2), which is a crystalline compound, by treating with a hydroacid in an organic solvent.

When crystallizing compound (2) (hydrohalic acid salt of (S)-α-halomethyl-3-pyridine-methanol derivative), crystallizing methods such as crystallization by cooling and crystallization by concentration can be used or these crystallizing methods can be used in combination. Crystallization by concentration can be a crystallization method wherein a solution comprising an organic solvent is replaced with a solution comprising another organic solvent. Also, when crystallizing, a seed crystal can be added.

From the viewpoints of crystallinity of the hydrohalic acid salt of (S)-α-halomethyl-3-pyridine-methanol derivative represented by formula (2) and ease in handling, examples of the hydrohalic acid used in this step are hydrofluoric acid, hydrochloric acid, hydrobromic acid and hydriodic acid and hydrochloric acid is preferable. To add the hydrohalic acid, a hydrohalic acid solution prepared by dissolving a hydrohalic acid in a suitable solvent in advance can be used or hydrohalic acid can be added directly to the organic solvent in which compound (1) ((S)-α-halomethyl-3-pyridine-methanol derivative) is dissolved.

From the viewpoints of drying of the solvent from the wet crystal and recovery and reuse of the solvent (distillation recovery), the organic solvent used in this step is preferably an organic solvent having a relatively low boiling point. Examples of such organic solvents are those usually having a boiling point of approximately at most 100°C under pressure of at most 1 atmosphere. The organic solvent is not particularly limited and examples are hydrocarbon solvents such as pentane, petroleum ether, neopentane, hexane, cyclohexane, methylcyclohexane, heptane, cycloheptane, octane, isooctane, nonane, decane, benzene, toluene, o-xylene, m-xylene, p-xylene, ethylbenzene, propylbenzene, cumene, n-butylbenzene and 1,3,5-mesitylene; ester solvents such as ethyl formate, methyl acetate, ethyl acetate, n-propyl acetate, n-butyl acetate, tert-butyl acetate, methyl propionate, ethyl propionate and γ-butyrolactane; ether solvents such as tert-butyl methyl ether, diethyl ether, diisopropyl ether, di-n-butyl ether, dimethoxyethane, diethylene glycol dimethyl ether, tetrahydrofurane acetone, tetrahydrofurane, 1,4-dioxane, anisole and acetone; ketone solvents such as methyl ethyl ketone, diethyl ketone, cyclopentanone and cyclohexanone; nitrile solvents such as acetonitrile and propionitrile; halogen solvents such as methylene chloride, chloroform, carbon tetrachloride, 1,2-dichloroethane, 1,1,1-trichloroethane and chlorobenzene and alcohol solvents such as methanol, ethanol, n-propanol, isopropanol and n-butanol.

More preferable examples are ester solvents such as ethyl formate, methyl acetate, ethyl acetate, n-propyl acetate, n-butyl acetate, tert-butyl acetate, methyl propionate, ethyl propionate and γ-butyrolactane and ether solvents such as tert-butyl methyl ether, diethyl ether, diisopropyl ether, di-n-butyl ether, dimethoxyethane, diethylene glycol dimethyl ether and tetrahydrofurane acetone. Furthermore, from an overall viewpoint of solvent cost and handling properties, methanol, ethyl acetate, tert-butyl methyl ether, hexane and toluene are most preferable. These may be used alone or two or more kinds can be used together.

When an organic solvent is used, high purification effect of compound (2) (hydrohalic acid salt of (S)-α-halomethyl-3-pyridine-methanol derivative) can be obtained. That is, effective removal of various impurities, particularly related substances of compound (1) ((S)-α-halomethyl-3-pyridine-methanol derivative), can be achieved. The amount of the organic solvent is preferably an amount by which flowability of the obtained substance can be obtained when the crystallization process of compound (2) is finished. For example, the amount is preferably approximately at most 30 times by weight, more preferably approximately 1 to 30 times by weight, most preferably 1 to 10 times by weight, based on compound (2).

In the present invention, when crystallizing, an auxiliary solvent can also be used besides the organic solvent, in order to improve at least one of yield of compound (2) (hydrohalic acid salt of (S)-α-halomethyl-3-pyridine-methanol derivative), concentration of' the treatment or properties of the obtained crystal. The auxiliary solvent can be added to the organic solvent when necessary and compound (2) can be dissolved by heating in advance into a mixture solution of the auxiliary solvent and the organic solvent and then crystallized by cooling.

The auxiliary solvent is not particularly limited and examples are hydrocarbon solvents such as pentane, petroleum ether, neopentane, hexane, cyclohexane, methylcyclohexane, heptane, cycloheptane, octane, isooctane, nonane, decane, benzene, toluene, o-xylene, m-xylene, p-xylene, ethylbenzene, cumene, n-butylbenzene and 1,3,5-mesitylene; ester solvents such as ethyl formate, methyl acetate, ethyl acetate, n-propyl acetate, n-butyl acetate, tert-butyl acetate, methyl propionate, ethyl propionate and γ-butyrolactane; ether solvents such as tert-butyl methyl ether, diethyl ether, diisopropyl ether, di-n-butyl ether, dimethoxyethane, diethylene glycol dimethyl ether and tetrahydrofurane acetone; ketone solvents such as methyl ethyl ketone, diethyl ketone, cyclopentanone and cyclohexanone; nitrile solvents such as acetonitrile and propionitrile; halogen solvents such as methylene chloride, chloroform, carbon tetrachloride, 1,2-dichloroethane, 1,1,1-trichloroethane. These may be used alone or two or more kinds can be used together.

Of these, from an overall viewpoint of drying of the solvent from the wet body, recovery and reuse of the solvent (distillation recovery) and cost and handling properties of the solvent, hexane, toluene or tert-butyl methyl ether are preferable.

The suitable amount of the auxiliary solvent can be determined by simple experiments. From the viewpoint of yield and flowability of the crystal slurry, the amount of the auxiliary solvent is an amount by which the volume ratio of the organic solvent and the auxiliary solvent (organic solvent/auxiliary solvent) becomes at most 20, when the crystallization process of compound (2) (hydrohalic acid salt of (S)-α-halomethyl-3-pyridine-methanol derivative) is finished. More preferably, an amount by which the ratio becomes at most 10 is used. The lower limit is not particularly limited, but in view of economical efficiency, the lower limit is usually at least 0.05, preferably at least 0.1. When the volume ratio of the organic solvent and the auxiliary solvent is more than 20, the volume of the auxiliary solvent is too little and the effects of adding may not be sufficiently obtained.

The purification process and isolation process of the present invention can be conducted near room temperature and when necessary, heating or cooling can be conducted. For example, when a common solvent is used, isolation and purification are conducted at approximately at most 60°C, usually -30°C to 50°C, preferably 0°C to 40°C, for the reason that the temperature of the solvent does not exceed the boiling point under pressure of 1 atmosphere.

Compound (2) obtained in this way is subjected to solid-liquid separation and when necessary, is also cake washed and dried. The method for solid-liquid separation is not particularly limited and examples are pressure filtration, filtration under reduced pressure and centrifugation. As the drying method, thermal decomposition or melting is avoided and drying under reduced pressure (vacuum drying) at approximately at most 60°C is preferable.

Hereinafter, the present invention is described in detail based on Examples but not limited thereto.

EXAMPLE 1

Preparation of (S)-N-(5-(2-chloro-1-hydroxyethyl)pyridine-2-yl)acetoamide

Various yeast strains were inoculated in 30 ml of a semisynthetic medium (glucose 40 g, yeast extract 3 g, $KH_2PO_4$ 1 g, $(NH_4)_2HPO_4$ 6.5 g, $MgSO_4 \cdot 7H_2O$ 0.8 g, $ZnSO_4 \cdot 7H_2O$ 0.006 g, $FeSO_4 \cdot 7H_2O$ 0.09 g, $CuSO_4 \cdot 7H_2O$ 0.005 g, $MnSO_4$ 0.001 g, NaCl 0.1 g, $CaCO_3$ 5 g, water 1 L, pH 6.8), which was sterilized in a 500 ml Sakaguchi flask, and aerobically cultured by shaking at 30°C for 2 days. A medium-sized test tube was charged with 1 ml of the obtained culture solution, 5 mg of N-(5-(2-chloro-acetyl)pyridine-2-yl)acetoamide and 40 mg of glucose and reaction was conducted by shaking at 30°C for 1 day. After the reaction was finished, the obtained reaction solution was extracted by ethyl acetate and concentrated under reduced pressure, to obtain a crude product containing the title compound. The conversion ratio was found by HPLC analysis of the crude product and the optical purity was found by HPLC analysis after the crude product was purified by thin-layer chromatography. The results are shown in Table 1.

Conversion ratio (%) =

amount of product/(amount of substrate + amount of product) × 100

HPLC analysis conditions

Column: Develosil ODS-HG-3 (made by Nomura Chemical Co., Ltd.)
Eluent: acetonitrile/0.1 % $KH_2PO_4$ = 25/75
Flow rate: 1.0 ml/min
Column temperature: 40°C
Detection wavelength: 210 nm

Optical Purity (%ee) = (S-R)/(S+R) × 100

(S and R represent the amount of enantiomers)
HPLC analysis conditions

Column: Chiralcel OJ (made by Daicel Chemical Industries, Ltd.)
Eluent: hexane/iso-propanol = 9/1
Flow rate: 1.0 ml/min
Column temperature: 40°C
Detection wavelength: 210 nm

TABLE 1

| Microorganism | | | Conversion Ratio (%) | Optical Purity (%ee) |
|---|---|---|---|---|
| genus | species | No. | | |
| *Candia* | *etchellsii* | IFO 1229 | 17 | 86 |
| *Candia etchellsii* | | IFO 1942 | 22 | 88 |
| *Candia* | *galacta* | IFO 10031 | 41 | 87 |
| *Candia* | *lactis-condensi* | IFO 1286 | 14 | 72 |
| *Candia* | *versatilis* | IFO 1941 | 28 | 98 |
| *Candida* | *fennica* | CBS 6028 | 12 | 40 |
| *Candida* | *magnoliae* | IFO 0705 | 26 | 53 |
| *Clavispora* | *lusitaniae* | IFO 1019 | 8 | 6 |
| *Cryptococcus* | *humicola* | CBS 5958 | 49 | 100 |
| *Debaryomyces* | *carsonii* | IFO 0795 | 25 | 82 |
| *Debaryomyces* | *hansenii* var. *hansenii* | IFO 0032 | 7 | 58 |
| *Geotrichum* | *candidum* | CBS 187.67 | 41 | 76 |
| *Hyphpopichia* | *burtonii* | IFO 0844 | 26 | 96 |
| *Metschnikowia* | *pulcherrima* | IFO 0561 | 18 | 10 |
| *Ogataea* | *polymorpha* | IFO 1475 | 31 | 91 |
| *Pachysolen* | *tannophilus* | IFO 1007 | 14 | 55 |
| *Pichia* | *anomala* | IFO 0707 | 28 | 23 |
| *Rhodotorula* | *araucariae* | IFO 10053 | 8 | 74 |
| *Rhodotorula* | *minuta* | IFO 0928 | 13 | 58 |
| *Saccharomyces* | *bayanus* | IFO 0676 | 4 | 54 |
| *Trichosporon* | *aquatile* | ATCC 22310 | 7 | 86 |
| *Trigonopsis* | *variabilis* | IFO 0671 | 32 | 21 |
| *Waltomyces* | *lipofer* | IFO 0673 | 39 | 94 |
| *Yamadazyma* | *farinosa* | IFO 0459 | 22 | 44 |

TABLE 1 (continued)

| Microorganism | | | Conversion Ratio (%) | Optical Purity (%ee) |
|---|---|---|---|---|
| genus | species | No. | | |
| *Yarrowia* | *lipolytica* | IFO 1542 | 29 | 15 |

EXAMPLE 2

Preparation of (S)-α-chloromethyl-3-pyridine-methanol

Recombinant E. coli HB101 (pNTS1G) accession number FERM BP-5835 (February 24, 1997, International Patent Organism Depositary, National Institute of Advanced Industrial Science and Technology, address: AIST Tsukuba Central 6, 1-1, Higashi 1-Chome, Tsukuba-shi, Ibaraki-ken 305-8566 Japan) was inoculated in 50 ml of 2xYT medium (tryptone 16 g, yeast extract 10 g, sodium chloride 5 g, water 1 L, pH before sterilization 7.0), which was sterilized in a 500 ml Sakaguchi flask, and cultured 'by shaking at 37°C for 18 hours. After 40 mg of $NADP^+$ and 56.1 g of glucose were added to 800 ml of the obtained culture solution, 40 g of 3-(2-chloro-acetyl)pyridine hydrochloride was added over 5 hours while adjusting the pH to 6.5 at 30°C and then agitation was conducted for 2 hours. After the reaction was finished, the reaction solution was extracted by ethyl acetate and concentrated under reduced pressure, to obtain a 31.4 g of yellow oily matter, which is the title compound. The chemical purity and optical purity were analyzed in the same manner as in Example 1. The chemical purity was 97.8 % and the optical purity was 99.8 %ee.
$^1H$-NMR ($D_2O$,400MHz/ppm); 3.62 (2H,bs), 4.66 (2H,s), 5.88 (1H,s)

EXAMPLE 3

Preparation of (S)-α-chloromethyl-3-pyridine-methanol

Recombinant E. coli HB101 (pNTFPG) accession number FERM BP-7117 (April 11, 2000, International Patent Organism Depositary, National Institute of Advanced Industrial Science and Technology, address: AIST Tsukuba Central 6, 1-1, Higashi 1-Chome, Tsukuba-shi, Ibaraki-ken 305-8566 Japan) was inoculated in 50 ml of 2xYT medium, which was sterilized in a 500 ml Sakaguchi flask, and cultured by shaking at 37°C for 18 hours. After 2 mg of $NAD^+$ and 2.8 g of glucose were added to 40 ml of the obtained culture solution, 2 g of 3-(2-chloro-acetyl)pyridine hydrochloride was added over 5 hours while adjusting the pH to 6.5 at 30°C and then agitation was conducted for 2 hours. After the reaction was finished, the reaction solution was extracted by ethyl acetate and concentrated under reduced pressure to obtain 1.6 g of yellow oily matter, which is the title compound. The chemical purity and optical purity were analyzed in the same manner as in Example 1. The chemical purity was 96.5 % and the optical purity was 99.0 %ee.

EXAMPLE 4

Isolation and purification of (S)-α-chloromethyl-3-pyridine-methanol hydrochloride

26.3 g of (S)-α-chloromethyl-3-pyridine-methanol obtained in Example 2 was dissolved in 250 ml of hydrogen chloride-methanol solution-10 (available from Tokyo Kasei Kogyo Co., Ltd.) and condensation was conducted under reduced pressure until the amount of liquid became 90 ml. The solution was stirred while slowly dropping 270 ml of tert-butyl methyl ether thereto, to form a slurry solution of (S)-α-chloromethyl-3-pyridine-methanol hydrochloride, and then by filtering and drying the solution under reduced pressure, 28.6 g of white crystal, which is the title compound, was obtained. The chemical purity and optical purity of (S)-α-chloromethyl-3-pyridine-methanol hydrochloride were analyzed in the same manner as in Example 1. The chemical purity was 99.8 % and the optical purity was at least 99.9 %ee. $^1H$-NMR ($D_2O$,400MHz/ppm); 3.62 (2H,bs), 4.66 (2H,s), 5.88 (1H,s)

EXAMPLE 5

Isolation and purification of (S)-α-chloromethyl-3-pyridine-methanol hydrochloride

1.5 g of (S)-α-chloromethyl-3-pyridine-methanol obtained in Example 3 was dissolved in 12 ml of hydrogen chloride-methanol solution-10 (available from Tokyo Kasei Kogyo Co., Ltd.) and condensation was conducted under reduced pressure until the amount of liquid became 4 ml. The solution was stirred while slowly dropping 12 ml of tert-butyl methyl ether thereto, to form a slurry solution of (S)-α-chloromethyl-3-pyridine-methanol hydrochloride' and then by filtering and drying the solution under reduced pressure, 1.4 g of white crystal, which is the title compound, was obtained. The chemical purity and optical purity of (S)-α-chloromethyl-3-pyridine-methanol hydrochloride were analyzed in the same manner as in Example 1. The chemical purity was 99.7 % and the optical purity was at least 99.9 %ee.

INDUSTRIAL APPLICABILITY

According to the process of the present invention, a (S)-α-halomethylpyridine-methanol derivative, which is useful as an intermediate of pharmaceutical products, can be easily prepared from inexpensive raw materials.

## Claims

1. A (S)-α-halomethyl-3-pyridine-methanol derivative represented by formula (1):

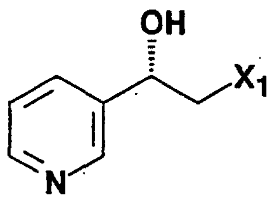

(1)

(wherein $X_1$ represents a halogen atom).

2. The compound of Claim 1, wherein $X_1$ is a chlorine atom.

3. A hydrohalic acid salt of a (S)-α-halomethyl-3-pyridine-methanol derivative represented by formula (2):

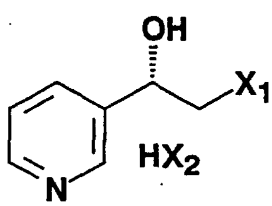

(2)

(wherein $X_1$ and $X_2$ represent a halogen atom).

4. The compound of Claim 3, wherein $X_1$ and $X_2$ are a chlorine atom.

5. A process for preparing a (S)-α-halomethylpyridine-methanol derivative represented by formula (4):

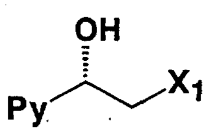

(4)

(wherein $X_1$ represents a halogen atom),
which comprises (S)-selectively reducing a 2-haloacetylpyridine derivative represented by formula (3):

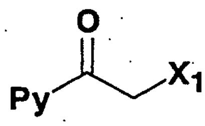

(3)

(wherein $X_1$ represents a halogen atom and Py represents a pyridyl group which can have a substituent) using an enzyme having ability to (S)-selectively reduce a carbonyl group of said 2-haloacetylpyridine derivative.

**6.** The process of Claim 5, wherein $X_1$ is a chlorine atom.

**7.** The process of Claim 5 or 6, wherein Py is a 3-pyridyl group, which can have a substituent.

**8.** The process of Claim 5, 6 or 7, wherein said substituent of said pyridyl group is at least one member selected from the group consisting of a nitro group, an N-protected amino group, a halogen atom and an alkoxy group having 1 to 10 carbon atoms.

**9.** The process of Claim 5, 6, 7 or 8, wherein said reducing enzyme having ability to (S)-selectively reduce a carbonyl group of said 2-haloacetylpyridine derivative is an enzyme present in a culture or a treated substance of a microorganism selected from the group consisting of Candida genus, Clavispora genus, Cryptococcus genus, Debaryomyces genus, Geotrichum genus, Hyphopichia genus, Metschnikowia genus, Ogataea genus, Pachysolen genus, Pichia genus, Rhodotorula genus, Saccharomyces genus, Trichosporon genus, Trigonopsis genus, Waltomyces genus, Yamadazyma genus and Yarrowia genus;
and/or a purified reducing enzyme derived from said microorganism.

**10.** The process of Claim 9, wherein said enzyme having ability to (S)-selectively reduce a carbonyl group of said 2-haloacetylpyridine derivative is an enzyme present in a culture or a treated substance of a microorganism selected from the group consisting of Candida etchellsii, Candida galacta, Candida lactis-condensi, Candida versatilis, Candida fennica, Candida magnoliae, Candida maris, Clavispora lusitaniae, Cryptococcus humicola, Debaryomyces carsonii, Debaryomyces hansenii var. hansenii, Geotrichum candidum, Hyphopichia burtonii, Metschnikowia pulcherrima, Ogataea polymorpha, Pachysolen tannophilus, Pichia anomala, Rhodotorula araucariae, Rhodotorula minuta, Saccharomyces bayanus, Trichosporon aquatile, Trigonopsis variabilis, Waltomyces lipofer, Yamadazyma farinosa and Yarrowia lipolytica;
and/or a purified reducing enzyme derived from said microorganism.

**11.** The process of Claim 9, wherein said enzyme having ability to (S)-selectively reduce a carbonyl group of said 2-haloacetylpyridine derivative is an enzyme present in a culture or a treated substance of a transformant of a microorganism selected from the group consisting of Candida genus, Clavispora genus, Cryptococcus genus, Debaryomyces genus, Geotrichum genus, Hyphopichia genus, Metschnikowia genus, Ogataea genus, Pachysolen genus, Pichia genus, Rhodotorula genus, Saccharomyces genus, Trichosporon genus, Trigonopsis genus, Waltomyces genus, Yamadazyma genus and Yarrowia genus, which is transformed by a gene of a reducing enzyme.

**12.** The process of Claim 11, wherein said transformant is Escherichia coli HB101 (pNTS1G) accession number FERM BP-5835 or Escherichia coli HB101 (pNTFPG) accession number FERM BP-7117.

**13.** A process for isolating and purifying, which comprises obtaining the compound represented by formula (1):

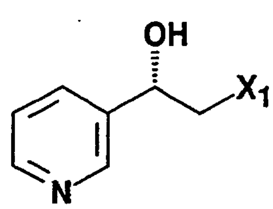

(1)

(wherein $X_1$ represents a halogen atom)

as crystal of a hydrohalic acid salt of a (S)-α-halomethyl-3-pyridine-methanol derivative represented by formula (2):

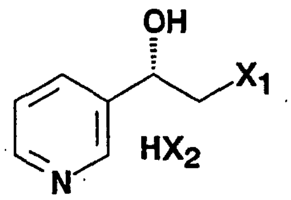

(2)

(wherein $X_1$ and $X_2$ represent a halogen atom)
from a solution containing said (S)-α-halomethyl-3-pyridine-methanol derivative represented by formula (1) and impurities using a hydrohalic acid salt represented by formula (5):

$$X_2H \qquad (5)$$

(wherein $X_2$ represents a halogen atom) and an organic solvent.

**14.** The process for isolating and purifying of Claim 13, wherein said (S)-α-halomethyl-3-pyridine-methanol derivative represented by formula (1) is prepared by the process of Claim 5, 6, 7, 8, 9, 10, 11 or 12.

**15.** The process for isolating and purifying of Claim 13 or 14, wherein $X_1$ is a chlorine atom.

**16.** The process for isolating and purifying of Claim 13, 14 or 15, wherein $X_2$ is a chlorine atom.

**17.** The process for isolating and purifying of Claim 13, 14, 15 or 16, wherein said impurities are enantiomers of said (S)-α-halomethyl-3-pyridine-methanol derivative represented by formula (1).

**18.** The process for isolating and purifying of Claim 13, 14, 15, 16 or 17, wherein said organic solvent is at least one member selected from the group consisting of a hydrocarbon solvent, an ester solvent, an ether solvent, a ketone solvent, a nitrile solvent, a halogen solvent and an alcohol.

**19.** The process for isolating and purifying of Claim 18, wherein said organic solvent is at least one member selected from the group consisting of pentane, hexane, cyclohexane, methylcyclohexane, heptane, octane, isooctane, benzene, toluene, ethylbenzene, propylbenzene, o-xylene, m-xylene, p-xylene, methyl' acetate, ethyl acetate, n-propyl acetate, n-butyl acetate, tert-butyl acetate, methyl propionate, tert-butyl methyl ether, diethyl ether, diisopropyl ether, di-n-butyl ether, tetrahydrofurane, 1,4-dioxane, anisole, acetone, methyl ethyl ketone, diethyl ketone, acetonitrile, propionitrile, methylene chloride, chloroform, carbon tetrachloride, 1,2-dichloroethane, chlorobenzene, methanol, ethanol, n-propanol, isopropanol and n-butanol.

## INTERNATIONAL SEARCH REPORT

International application No.
PCT/JP02/10730

A. CLASSIFICATION OF SUBJECT MATTER
Int.Cl$^7$ C07D213/30

According to International Patent Classification (IPC) or to both national classification and IPC

B. FIELDS SEARCHED

Minimum documentation searched (classification system followed by classification symbols)
Int.Cl$^7$ C07D213/30

Documentation searched other than minimum documentation to the extent that such documents are included in the fields searched

Electronic data base consulted during the international search (name of data base and, where practicable, search terms used)
CAPLUS(STN), REGISTRY(STN), WPIL(QUESTEL)

C. DOCUMENTS CONSIDERED TO BE RELEVANT

| Category* | Citation of document, with indication, where appropriate, of the relevant passages | Relevant to claim No. |
|---|---|---|
| P,X | WO 02/06258 A1 (American Home Products Corp.), 24 January, 2002 (24.01.02), Page 58; examples 30, 31 & US 2002/032222 A1 & US 6465501 B2 | 1 |
| X | WO 00/48997 A1 (Kaneka Corp.), 24 August, 2000 (24.08.00), Claims 18 to 26; page 20, lines 2 to 10; pages 40 to 42; examples 10, 11 & EP 1153919 A1 | 1-19 |
| A | WO 98/21184 A1 (PFIZER INC.), 22 May, 1998 (22.05.98), & AU 9746346 A1 & EP 938476 A & CN 1237160 A & BR 9712951 A & JP 12-504347 A & ZA 9710186 A & US 6291489 B1 & NO 9902296 A & KR 2000053314 A | 1-19 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 January, 2003 (21.01.03) | 04 February, 2003 (04.02.03) |
| Name and mailing address of the ISA/ Japanese Patent Office | Authorized officer |
| Facsimile No. | Telephone No. |

Form PCT/ISA/210 (second sheet) (July 1998)

INTERNATIONAL SEARCH REPORT

International application No.
PCT/JP02/10730

C (Continuation). DOCUMENTS CONSIDERED TO BE RELEVANT

| Category* | Citation of document, with indication, where appropriate, of the relevant passages | Relevant to claim No. |
|---|---|---|
| A | WO 99/32476 A1 (Bayer Corp.), 01 July, 1999 (01.07.99), & CA 2316971 A & AU 9918300 A & EP 1040106 B1 & BR 9814302 A & JP 13-526282 A & AT 222901 A & NO 2000003083 A | 1-19 |
| A | WO 97/16189 A1 (Merck and Co., Inc.), 09 May, 1997 (09.05.97), & AU 9674845 A & EP 858340 A1 & JP 11-515027 A | 1-19 |

Form PCT/ISA/210 (continuation of second sheet) (July 1998)